# EUROPEAN PATENT APPLICATION

(11) **EP 2 505 633 A1**
(43) Date of publication of application: **03.10.2012**
(21) Application number: 11160293.4
(22) Date of filing: 29.03.2011
(51) Int. Cl.: C12M 1/00, C12M 1/02

(54) **Photobioreactor for growing organisms**

(71) Applicant: Algae Health, Claregalway Galway (IE)
(72) Inventor: McHugh, Edward, Co. Galway (IE)
(74) Representative: Lane, Cathal Michael

(57) **Abstract**

A photo bioreactor for the growth of photosynthetic organisms comprising a tank (2) for holding a liquid medium, six rotational agitators (3a-3f) for agitating the liquid medium. Each agitator is arranged to agitate about a substantially vertical axis (6a-6f) by sweeping out, upon rotation, a substantially circular path (C) that has a diameter (D) that is substantially coincidental with a radius (R) of the tank (2). Each agitator (3a-3f) is located on a radius which is spaced approximately 60⁰ apart from the next. This allows for more even mixing and more uniform light distribution within the tank.

## Description

### Field of the Invention

The present invention relates to growing organisms. In particular the present invention relates to the growth of organisms using light. Organisms that grow using light will be referred to as photosynthetic or phototropic organisms. Of particular interest is the growth of algae for example macroalgae such as seaweed and microalgae. The present invention is concerned with the provision of suitable equipment to allow growth of organisms, in particular microorganisms such as algae, bacteria such as cyanobacteria and the like using light.

### Background to the Invention

Technology for the cultivation of organisms, and in particular photosynthetic organisms, has been known for many years and there has been a significant level of advancement in the area. These include optimisation of cultivation conditions, the provision/identification of new strains of organism and in particular algae, and the development of equipment for growing the organisms. Work has also been done to determine the level of stress that can be imparted to the organism to optimise yields of specific extracts formed within the organism. The organisms are typically grown for harvesting. Extracts, for example compounds with specific end uses may be extracted from them.

However, there has been an issue with the commercialisation of growing photosynthetic organisms. This is because it has been difficult to obtain commercially viable yields in traditional growing systems. For example such organisms have often been grown in an open pond where ambient conditions prevail and where it is thus difficult to optimise growth conditions.

Assuming that other conditions for optimal growth of the organisms are known, for example nutrient levels, temperature and pH, then the limiting factor on growth tends to be the provision of suitable light and in particular suitable quantities and intensities of light.

As a result there been a move to photo bioreactors. These bioreactors are various forms of equipment that are intended to improve growth conditions, particularly by increasing exposure of the organisms to light. One typical configuration of photo bioreactor is an array of light transparent tubes through which a suitable liquid medium carrying the organism is circulated by pumping. Often the medium is water-based and the mix forms a suitable culture for growth of the organism. The culture is exposed to light as it travels through the light transparent tubes. By providing tubes of relatively small diameter, light penetration is increased. However, as the culture becomes denser with the growth of the organism light penetration becomes more limited and thus the efficiency of such photo bioreactors decreases. In practice it has been found that such photo bioreactors do not produce growth rates sufficient and/or extract yields to make the operation commercially sustainable. While they may be just about sufficiently efficient to justify the production of high value extracts they typically are not suitable for lower cost products. While the system has the advantage of using natural light it has disadvantages which include limited light penetration (in particular as culture density increases), variation in light intensity and duration (as the light is ambient light), high capital set-up cost and low-volume relative to the area occupied. Other problems include oxygen build up within the system (due to the photosynthesis process of the organisms) which may impair growth and potential shear stresses imparted due to pumping which can physically damage the organisms. International Patent Publication WO 2010/142870 shows a system based on such an arrangement.

Some attempts have been made to provide alternative photo bioreactors that overcome some of the issues discussed above.

Such alternatives include tanks which are illuminated from the outside by shining artificial light through transparent walls of the tank or through windows defined in the tank.

Other systems provided include illumination systems which are intended to be placed in a tank in which the organism is being grown. For example International Patent Publication WO 2010/115655 describes a plurality of light tubes each with an array of lights which are to be arranged inside the volume of a growth tank. The light tubes are positioned equidistantly with respect to each other and are said to provide substantially homogenous illumination of the inside of the container. However, it is clear that in a system that has fixed lighting that organisms closest to the lighting will receive greater exposure to light than those which is more distant from the light source. There is thus the possibility of organisms closer to the light source being damaged due to overexposure to light while those furthest away may not receive enough light stimulation to grow. Accordingly, at best there will be an uneven growth pattern within the tank, and at worst no growth in some areas and light damage in other areas. US Patent Publication No. 2010/0323436 describes a tank system with a convoluted flow-through path for the liquid medium. International Patent Publication WO 2010/117720 describes a photo bioreactor formed by joining upper and lower layers of transparent film and with flow channels defined therein.

International Patent Publication WO 2009/142765 describes a system for growing photosynthetic organisms which includes a tank in which a rotatable light array is placed. The light array comprises a central axle which is orientated vertically in the tank and which has a series of horizontal and parallel arms thereon. Two or more arrays can be provided so that the light arrays intermesh upon rotation. Japanese Patent Publication No. 2008283937 describes a similar system. US Patent No. 7,824,904 describes a photo bioreactor in which a rotating mixer is located within a tank. A light source outside the tank transmits light to a light-emitting device on the blades of the mixer. While such systems will have better light distribution properties than a fixed system such as that described above, there is still a lack of efficiency within these systems. The system still require significant improvement to enable commercially viable production of desired product.

Many other systems have also been described. They include the system described in US Patent No. 4,952,511 which describes a photo bioreactor for the cultivation of photosynthetic microorganisms. It includes a tank, a plurality of light compartments in the form of tubes extending into the tank, and high intensity lamps whose light is directed into the tubes. Each tube has a transparent wall and means for distributing light from a lamp substantially uniformly across the transparent wall. US Patent Publication No. 2010/0144019 shows a series of light tubes which extend downward into a tank and further including a mechanical stirrer and a pneumatic mixing device. The stirrer creates liquid circulation. The pneumatic mixing device generates bubbles to suspend microalgae in the culture medium.

Notwithstanding the foregoing systems it is still desirable to provide an alternative system for photosynthetic growth of organisms, and in particular microorganisms, within a liquid medium. The present invention set out to overcome the inefficient systems described above and to provide greater efficiency. This allows for better commercial viability of production of the organisms and in turn products made from them or extracted from them.

### Summary of the Invention

The present invention provides a photo bioreactor for the growth of photosynthetic organisms comprising:
(i) a growth receptacle for holding a liquid medium;
(ii) at least three rotational agitators located within the growth receptacle for agitating the liquid medium;
(iii) wherein each agitator is arranged to agitate about a substantially vertical axis by sweeping out, upon rotation, a substantially circular path that has a diameter that is substantially coincidental with a radius of the receptacle.

This provides a simple yet highly effective system for agitation of photosynthetic organisms in a tank. Importantly it also provides for even distribution of light with the tank where light shines into the tank from each agitator. The vertical axis for each agitator is independent of the others and in particular each vertical axis is spaced apart from the next.

Desirably at least two agitators rotate in opposing directions. This allows for better mixing and light distribution.

Desirably each agitator is coincidental with a radius of the receptacle, each radius being spaced approximately 120° apart from the next. This evenly spaced arrangement allows for even mixing, good agitation and good light distribution.

A photo bioreactor of the invention may comprise six agitators each being coincidental with a radius of the receptacle. For example each radius may be spaced approximately 60° apart from the next. Geometrically this has been found to be an optimal arrangement.

Desirably the agitators are substantially linear and suitably are each the same as the next in size and shape. Having uniform agitators is important for even mixing. Being substantially linear allows for them to sweep past each other without fouling each others movement.

A photo bioreactor of the invention may comprise an even number of agitators where every agitator rotates in an opposite direction to the next. Again from a mixing and even distribution of light point of view this is an advantageous arrangement.

A photo bioreactor may comprise an arrangement of agitators where the distance from the centre of one agitator to the centre of an adjacent one is approximately the same as one half of the length of the radius of the receptacle. This even spacing allows for better interworking of the agitators.

Desirably each agitator is rotationally offset from the next, for example by about 90 degrees, so that a first agitator can rotate past a second at a position close to the vertical axis thereof without the agitators colliding.

At least one agitator and desirably each agitator may have an agitator body with a substantially continuous surface that is substantially the same height as the tank. This allows for mixing and light distribution throughout the depth of the tank. At least one agitator and desirably each agitator may have an agitator body with a substantially continuous surface that is substantially the same length as a radius of the tank. That is the agitator extends in a radial direction for a length that is substantially the same as a radius of a tank.

It is desirable that an agitator extends for substantially all of a radial length of and substantially all of the height of the tank and in particular it is desirable that the agitator body is substantially continuous in both dimensions. Desirably the propeller is substantially linear, for example with an agitator blade or paddle on each of opposing sides of a pivot axis. With a linear arrangement there is better ability to have the agitators cross into and out of each others path without obstructing each other.

For growth of photosynthetic organism it is desirable that each agitator has an agitator body which emits light for promoting growth of photo synthetic organisms. For example each agitator may have an agitator body that is made of a substantially light transparent material. Alternatively the agitator body may comprise, for example may be covered with, a light emitting material.

The invention extends to a photo bioreactor substantially as described herein with reference to and as illustrated in the accompanying drawings.

### Brief Description of the Drawings

Embodiments of the invention will be described, by way of example only, with reference to the accompanying drawings in which:

**Figure 1** is a schematic representation from a top plan view perspective of a photo bioreactor of the present invention;

**Figure 2** is a similar view to that of Figure 1 but with a circle and a triangle superimposed on the representation to illustrate relative distances; and

**Figure 3** is a perspective view of the agitators from above and to one side of the photo bioreactor of Figure 1.

### Detailed Description of the Drawings

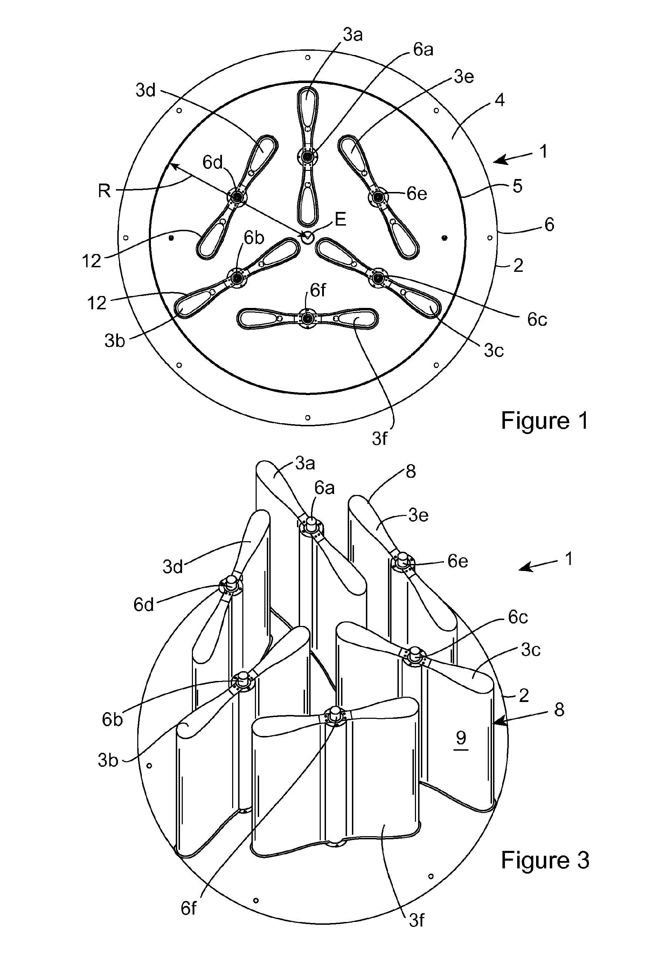

With reference to **Figures 1 to 3** there is shown a photo bioreactor 1 for the growth of photosynthetic organisms and comprising: a growth receptacle in the form of a tank 2 for holding a liquid medium. The tank 2 is circular in shape being defined by a substantially circular perimetric wall 4, with an inner wall surface 5 and an outer wall surface 6. The tank 2 has a centre point E.

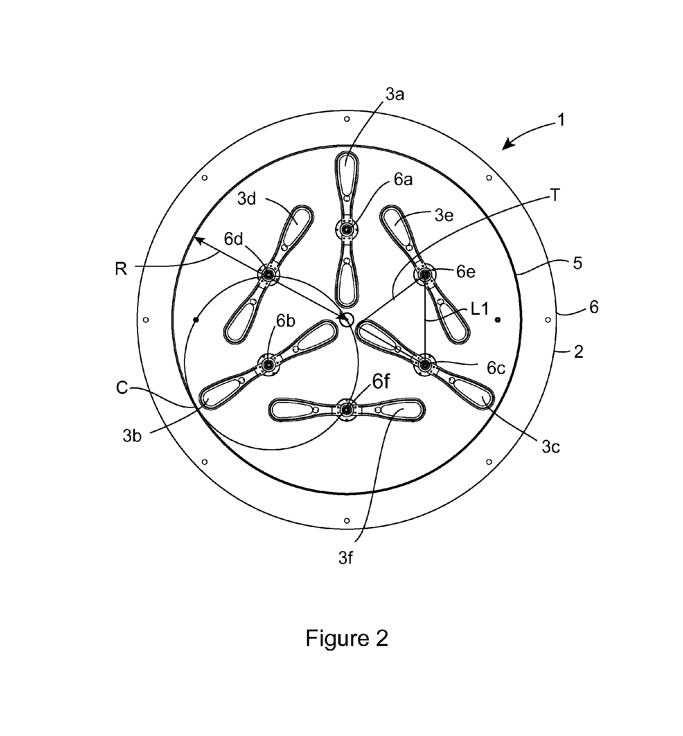

At least three and in particular six (6) rotational agitators 3a-f are located within the tank 2 for agitating the liquid medium. Each agitator 3a-3f is arranged to respectively agitate about a substantially vertical axis 6a-f by sweeping out, upon rotation, a substantially circular path C (see **Figure 2**) that has a diameter D that is substantially coincidental with a radius R of the receptacle. It will be appreciated that each agitator will rotate and align itself along the radius R during its cycle. In fact a linear agitator such as those shown will align itself upon the radius R at least once and in fact twice during its rotation. Each agitator 3a-3f is in length substantially the same length as the radial distance between the centre point C and the circumferential wall 4 of the tank 2. Each agitator can be considered to be linear with an agitator blade or paddle on each of opposing sides of its pivot axis.

Three agitators 3a, 3b and 3c are coincidental with a radius R of the receptacle and, as is desirable, where each radius is spaced approximately 120° apart from the next. This is achieved my mounting the agitators 3a,3b and 3c on respective vertical axes 6a-6c which are each positioned on a radius R of the tank 2 each of which is approximately equidistant from the next about the tank 2.

Indeed the photo bioreactor 1 has a second set of agitators 3d, 3e and 3f which are each coincidental with a radius R of the receptacle and where those radii are spaced approximately 120⁰ apart from the next.

Overall then the configuration is that there are six (6) agitators 3a-3f which are coincidental with a radius R of the receptacle and, as is desirable, where each radius is spaced approximately 60⁰ apart from the next. This gives the configuration shown in the Figures and provides for evenly distributed agitation of the tank 2. In particular it is thought that the arrangement of the present invention provides for maximum mixing as substantially all of the area and indeed volume of the tank 2 is swept by one or more of the agitators 3a-3f.

In the arrangement shown the distance L1 from the centre (rotational axis) of one agitator, for example agitator 3c to the centre of an adjacent one, for example 3e is approximately the same as one half of the length of the radius R of the receptacle.

In the configuration shown every agitator rotates in an opposite direction to the next adjacent one. So for example agitators 3a, 3b and 3c may move in one direction (e.g. clockwise) and each of agitators 3d, 3e and 3f may move in an opposite direction (e.g. anticlockwise). Again this provides for more thorough mixing.

As can also be seen from the Figures each agitator is rotationally offset from the next so that a first agitator (e.g. 3a) can rotate past a second (e.g. 3e) at a position close to the vertical axis (e.g.6e) thereof without the agitators 3a,3e colliding. Indeed each agitator (e.g. 3a) has two adjacent agitators (e.g. 3d and 3e) and each of the adjacent agitators (e.g. 3d,3e) moves past that agitator (e.g. 3a)close to its vertical axis (e.g.6a).

This means that liquid close to the centre of each agitator can be moved away by at least one of its neighbouring agitators.

As best seen from Figure 3 (where the wall 4 of the tank 2 has been removed for ease of illustration) each agitator 3a-3f has an agitator body 8 with a substantially continuous agitation (outer) surface 9 that is substantially the same height as that of the tank 2. Indeed that agitation surface 9 is substantially continuous along substantially the same length as a radius R of the tank. Indeed the agitator body 8 is substantially linear and may be considered to have a linear propeller type shape.

The agitator body 8 has defined therein hollow portions in the form of cavities 12. One or more light sources can be provided on each agitator 3a to 3f so that each has an agitator body which emits light for promoting growth of the photo synthetic organisms. For example each agitator 3a-3f may have an agitator body 8 that is made of a substantially light transparent material. Light can be directed into the agitator 3a-3f and emitted through the transparent walls. One or more light sources can be placed internally within the cavities 12 so as to emit light through the agitator body 8. Additionally or alternatively light sources may fixed in the agitator body for example where the agitator body 8 is not transparent. The agitator body may be covered in a light emitting material.

The design of the invention thus enables the spread of light evenly through a growth culture. This means that all phototropic organisms being grown continuously receives a uniform amount of light. It will be appreciated that in a single linear paddle system, for example where one paddle mixes the entire tank by being mounted with a rotational axis at the centre of the tank and being substantially the same length as a diameter of the tank, that the liquid culture will follow the agitator around. This means that because the agitator body close to the centre of the agitator moves a smaller distance relative to the agitator body toward the outer tips of the agitator there is uneven mixing and if light is emitted from the agitator an uneven exposure to light as the exposure is dependent on the distance from the centre of the agitator.

So the present invention shows that if an agitatior is pivoted from a centre point on a radius of circular tank with the agitator length being substantially equal to the length of the radius and the agitator being pivoted from it centre, then upon rotation, the agitator then will "touch" the centre of the tank out to the outside of the tank. In this respect "touch" means sweep past in close proximity. As the skilled person will appreciate it is not desirable for the agitators to rub against any part of each other or the tank.

If 6 agitators are positioned at 60 degrees apart, the distance from the centre of one agitator to the centre of the next closest agitator is also equal to half the radius of the tank. This is illustrated by the (equilateral) triangle T drawn in Figure 2. This means as the agitator rotates it will then touch the centre of the tank, the outside of the tank and the centre of both agitators beside it. The agitators are offset, for example to operate at a rotational separation of 90 degrees to the agitator beside them (so when a agitator is in line with the outside and centre of the tank, the adjacent agitator is set at 90 degrees so that they are pointing at the centre axis of the adjacent agitators). As the rotation of adjacent agitators is in opposite directions this will avoid a collision between agitators.

The effect of this is that biological material (e.g. microalgae) is continuously moved from being in contact with the tip of one agitator and as it is rotated it moves to the inside of the next agitator and *vice versa.* This provides all the biological material (e.g. microalgae) with a uniform quantity of light. The biological material is passed on from each agitation agitator as it is moved from the centre to agitator tip and from agitator to agitator.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A photo bioreactor for the growth of photosynthetic organisms comprising:
(a) a growth receptacle for holding a liquid medium;
(b) at least three rotational agitators located within the growth receptacle for agitating the liquid medium;
wherein:
each agitator is arranged to agitate about a substantially vertical axis by sweeping out, upon rotation, a substantially circular path that has a diameter that is substantially coincidental with a radius of the receptacle.

2. A photo bioreactor according to Claim 1 wherein at least two agitators rotate in opposing directions.

3. A photo bioreactor according to any preceding claim wherein each agitator is coincidental with a radius of the receptacle, each radius being spaced approximately 120⁰ apart from the next.

4. A photo bioreactor according to any preceding claim comprising six agitators each being coincidental with a radius of the receptacle

5. A photo bioreactor according to Claim 4 wherein each radius is spaced approximately 60⁰ apart from the next.

6. A photo bioreactor according to any preceding claim comprising an even number of agitators and wherein every agitator rotates in an opposite direction to the next.

7. A photo bioreactor according to any preceding claim comprising an arrangement of agitators where the distance from the centre of one agitator to the centre of an adjacent one is approximately the same as one half of the length of the radius of the receptacle.

8. A photo bioreactor according to any preceding claim wherein each agitator is rotationally offset from the next so that a first agitator can rotate past a second at a position close to the vertical axis thereof without the agitators colliding.

9. A photo bioreactor according to any preceding claim wherein each agitator has an agitator body with a substantially continuous surface that is substantially the same height as the tank.

10. A photo bioreactor according to any preceding claim wherein each agitator has an agitator body with a substantially continuous surface that is substantially the same length as a radius of the tank.

11. A photo bioreactor according to any preceding claim wherein each agitator has an agitator body which emits light for promoting growth of the photo synthetic organisms.

12. A photo bioreactor according to any preceding claim wherein at least one agitator has an agitator body that is made of a substantially light transparent material.

13. A photo bioreactor according to any preceding claim wherein at least one agitator has an agitator body that comprises a light emitting material.

14. A photo bioreactor substantially as described herein with reference to and as illustrated in the accompanying drawings.
